# EUROPÄISCHE PATENTSCHRIFT

(11) **EP 0 536 440 B1**
(45) Veröffentlichungstag und Bekanntmachung des Hinweises auf die Patenterteilung: **28.05.1997**
(21) Anmeldenummer: 91117332.6
(22) Anmeldetag: 11.10.1991
(51) Int. Cl.: A61B 17/39

(54) **Instrument für die Hochfrequenzchirurgie zum Schneiden oder Koagulieren**
H.F. surgical instrument for cutting and coagulating
Appareil de chirurgie à H.F. pour couper et coaguler

(43) Veröffentlichungstag der Anmeldung: 14.04.1993
(73) Patentinhaber: Erbe Elektromedizin GmbH., D-72072 Tübingen (DE)
(72) Erfinder: Buess, Gerhard, Professor Dr. med., W-7400 Tübingen (DE); Farin, Günter, W-7400 Tübingen-Hirschau (DE); Fischer, Klaus, W-7270 Nagold-Emmingen (DE)
(74) Vertreter: Endlich, Fritz, Dipl.-Phys. Patentanwalt

(56) Entgegenhaltungen:
- US-A- 2 888 928
- US-A- 4 307 720
- US-A- 4 326 529
- US-A- 5 007 908

## Beschreibung

Die Erfindung betrifft ein Instrument für die Hochfrequenzchirurgie zum Schneiden und/oder Koagulieren biologischer Gewebe mit HF-Strom entsprechend dem Oberbegriff des Patentanspruchs 1.

Ein Instrument dieser Art ist aus der US-A-4,043,342, bekannt, bei welchem eine dünne metallische Nadel als aktive Schneideelektrode in einer als Neutralelektrode wirkenden Metallhülse angeordnet ist, wobei der HF-Strom während Schneidevorgängen zwischen der Nadel und der Metallhülse durch das zu schneidende Gewebe fließt.

Das aus der US-A-4,043,342, bekannte Instrument ist zum Schneiden insofern vorteilhaft, als der HF-Strom nicht durch den Körper des Patienten fließen muß, sondern auf den Bereich zwischen der als Schneideelektrode wirkenden Metallnadel und der als Neutralelektrode wirkenden Metallhülse begrenzt ist. Da die HF-Stromdichte innerhalb der Kontaktfläche zwischen der als Neutralelektrode wirkenden Metallhülse und dem von dieser Metallhülse berührten Gewebe nicht zu groß sein darf, weil andernfalls unbeabsicht thermische Schädigungen des betreffenden Gewebes entstehen können, muß die als Schneideelektrode wirkende Metallnadel möglichst dünn sein, so daß der zum Schneiden erforderliche HF-Strom möglichst klein ist. Je dünner die Metallnadel jedoch ist, desto geringer ist die thermische Koagulationszone der Schnittflächen und deren blutstillende Wirkung. Zum partiellen Koagulieren bzw. Blutstillen ist dieses Instrument außerdem nicht geeignet. Entstehen beim Schneiden Blutungen, so muß der Chirurg ein anderes, zum Koagulieren geeignetes Instrument, verwenden. Dies ist insbesondere bei endoskopischen Operationen problematisch, weil das Wechseln der Instrumente zeitraubend ist. Bei stärkeren Blutungen kann außerdem die Sicht auf die Blutungsquelle durch das während des Instrumentenwechsels ausfließende Blut erschwert oder gar unmöglich werden. In diesen Situationen muß der Chirurg das ausgeflossene Blut beispielsweise mit Sauginstrumenten entfernen.

Für endoskopische Operationen sind Saugrohre bekannt (z.B. Koagulations-Saugrohr mit unipolarem HF-Anschluß, Artikel Nr. 8840.73 von R. WOLF GmbH, Katalog B 608/VII.88 ) welche auch zum Koagulieren bzw. Blutstillen geeignet sind. Zum Schneiden sind diese Koagulations-Saugrohre jedoch nicht geeignet.

Für endoskopische Operationen sind auch HF-Elektroden mit metallischem Präparierhaken bekannt, welche zum monopolaren Schneiden angewendet werden können und welche mit einem Saugkanal zum Absaugen von Rauch ausgestattet sind(z.B. Olympus Artikel Nr. A5601) . Da der metallische Präparierhaken zum mechanischen Präparieren mit Rücksicht auf unbeabsichtigte Verletzung des zu präparierenden Gewebes nicht zu dünn sein darf, ist der zum Schneiden mit diesem Präparierhaken erforderlich HF-Strom relativ groß. Dieser relativ große HF-Strom muß vom monopolaren Präparierhaken durch den Körper des Patienten zu einer auf der Haut des Patienten applizierten Neutralelektrode fließen. Dies ist insbesondere bei endoskopischen Operationen insofern problematisch, als der HF-Strom innerhalb des Körpers durch feine Gewebestrukturen fließen muß. Um unbeabsichtigte thermische Schädigungen von Gewebe zu vermeiden, sollen die zum Schneiden und/oder Koagulieren erforderlichen HF-Ströme jedoch möglichst klein sein.

Aus der US-PS 5,007,980 ist ein hochfrequenzchirurgisches Instrument mit einer nadelförmigen Elektrode bekannt, welche in einem Hohlraum des Instruments axial angeordnet ist. Die dort gezeigte Nadel bzw. der Draht ist axial beweglich, so daß das Instrument einmal zum Schneiden und andererseits zum Koagulieren verwendbar ist. Über einen Kanal, der der Aufnahme des Drahtes dient, kann eine Lösung zum Spülen der zu behandelnden Stelle des Gewebes zugeführt werden. Der Durchmesser des Kanals bzw. der entsprechenden Öffnung ist größer als der Außendurchmesser des verwendeten Drahtes, so daß die entsprechende Flüssigkeitsmenge bzw. Spüllösung strömen und austreten kann. Es hat sich jedoch gezeigt, daß insbesondere bei außerordentlich dünnen Drähten, die als Nadelelektrode wirken, eine nicht vorhandene Führung am distalen Ende des Instruments zu einer Instabilität des Drahtes führt, so daß die Schnitte nicht mit der erforderlichen Exaktheit durchführbar sind.

Es ist daher Aufgabe der Erfindung, ein Instrument für die Hochfrequenzchirurgie zum Schneiden und/oder Koagulieren biologischer Gewebe anzugeben, mit dessen Hilfe die gewünschten chirurgischen Eingriffe mit hoher Präzision und mit geringer Patientenbelastung durchgeführt werden können.

Die Lösung der Aufgabe der Erfindung erfolgt mit einem Gegenstand nach den Merkmalen des Patentanspruches 1, wobei die Unteransprüche mindestens zweckmäßige Ausgestaltungen und Weiterbildungen umfassen.

In bevorzugten Ausführungsformen gemäß der Erfindung ist eine Hülse an ihrem vom Patienten distalen Ende mit einem Schaft ausgestattet, welcher aus einem Rohr besteht und eine dem jeweiligen Anwendungszweck entsprechende Länge von beispielsweise von 5 bis 20 cm für Anwendungen in der offenen Chirurgie und 20 bis 50 cm bei endoskopischer Anwendung und einen Durchmesser von beispielsweise 5 bis 10 mm hat . Außerdem ist die Außenfläche der Hülse und des Schafts außen, exclusive der zum Patienten proximalen Stirnfläche der Hülse, mit einer elektrischen Isolierschicht ausgestattet. Die elektrisch nicht isolierte Stirnfläche der Hülse kann als Koagulationselektrode oder beim Schneiden mit der Nadelelektrode als Neutralelektrode angewendet werden. Die Hülse besteht beispielsweise aus einem Metallrohr, welches an der zum Patienten proximalen Stirnfläche konvex, beispielsweise halbkugelförmig, bis auf ein kleines Loch; aus welchem die Nadelelektrode herausgeschoben werden kann, geschlossen ist. Hülse und Schaft können auch einstückig aus einem Rohr geformt sein, wobei die Durchmesservon Hülse und Schaft ungleich oder auch gleich sein können. Der Durchmesser der Hülse sollte mit Rücksicht auf eine ungestörte Sicht auf die Nadelelektrode möglichst dünn sein. Der Durchmesser des Schaftes sollte mit Rücksicht auf die mechanische Stabilität des Instrumentes und eines innerhalb des Schafts anzuordnenden Saug- und/oder Spülkanals möglichst groß sein.

Innerhalb und/oder außerhalb von Hülse und/oder Schaft sind Saug- und/oder Spülkanäle angeordnet, welche am zum Patienten proximalen Ende der Hülse bzw. des Instruments in Saug- und/oder Spüllöchern und am vom Patienten distalen Ende des Schafts bzw. des Instruments in Anschlüssen für Saug- und/oder Spüleinrichtungen enden. Saug- und/oder Spüleinrichtungen sind vom erfindungsgemäßen Instrument unabhängige, ansich bekannte Einrichtungen und werden deswegen hier nicht weiter beschrieben. Die Saug- und/oder Spüllöcher sind beispielsweise in der Stirnfläche der Hülse angeordnet, wofür vorzugsweise auch das Loch in der Stirnfläche der Hülse, durch welches die Nadelelektrode herausgeschoben wird, verwendet werden kann wenn die Nadelelektrode in die Hülse zurückgezogen ist. Hierzu muß die Nadelelektrode so weit in die Hülse zurückziehbar sein, daß Gase und/oder Flüssigkeiten ungehindert in den Saug- und/oder Spülkanal bzw. Spülflüssigkeiten ungehindert aus dem Saug- und/oder Spülkanal auf das Operationsfeld fließen oder gespritzt werden können. Zusätzlich zu diesem Loch, durch welches die Nadelelektrode herausgeschoben werden kann, können weitere Saug- und/oder Spüllöcher, vorzugsweise im Bereich der Stirnfläche der Hülse angeordnet sein, durch welche bei hineingezogener Nadelelektrode zusätzliche oder bei herausgeschobener Nadelelektrode allein Gase oder Spülflüssigkeiten in das Operationsfeld appliziert oder Gase und/oder Flüssigkeiten aus dem Operationsfeld abgesaugt werden können.

In einer einfachen Ausführung eines erfindungsgemäßen Instruments ist nur ein Kanal innerhalb oder außerhalb von Hülse und/oder Schaft des Instruments vorhanden, welcher abwechselnd zum Saugen oder Spülen verwendet werden kann.

In einer erweiterten Ausführung eines erfindungsgemäßen Instruments sind zwei Kanäle innerhalb und/oder außerhalb von Hülse und/oder Schaft vorhanden, welche gleichzeitig oder abwechselnd zum Saugen und/oder Spülen verwendet werden können. Beide Kanäle können innerhalb und/oder außerhalb des Schafts und/oder der Hülse bis zur Hülse und/oder bis zu den Saug- und/oder Spüllöchern voneinander getrennt geführt werden, wo sie entweder separat in entsprechende Sauglöcher und Spüllöcher einmünden oder kurz vor dem zum Patienten proximalen Ende des Instruments vereinigt in mindestens eine gemeinsame Öffnung einmünden, durch welches abwechselnd Saugen oder Spülen möglich ist. Bei einer derartigen Ausführung ergibt sich zusätzlich der Vorteil, daß eine einfache Reinigung des Saugkanals möglich ist, indem das oder die gemeinsamen Öffnungen geschlossen werden und die Spülflüssigkeit innerhalb des Instruments kurz vor der geschlossenen Öffnung bzw. den geschlossenen Öffnungen aus dem Spülkanal in den Saugkanal hineingesaugt wird um den Saugkanal beispielsweise von Blut und anderen Gewebebestandteile zu reinigen.

Hülse, Nadelelektrode, Stirnfläche der Hülse sowie Saug- und/oder Spülkanäle können in der selben Achse wie der Schaft des Instruments ausgerichtet sein, was bei Anwendung dieses Instruments in der offenen Chirurgie aber auch bei einigen endoskopischen Anwendungen, wie beispielsweise bei der laparoskopischen Adhäsiolyse, zweckmäßig sein kann.

Um die Freiheitsgrade der Schnittführung und/oder der Applikation der als Koagulationselektrode anwendbaren Stirnfläche der Hülse und/oder die räumliche Ausrichtung der Saug- und/oder Spüllöcher insbesondere bei endoskopischer Anwendung, z.B. bei der laparoskopischen Cholecystektomie, bei der transanalen endoskopischen Mikrochirurgie oder bei der Adhäsiolyse flexibler zu gestalten, ist die Hülse inklusive Nadelelektrode und elektrischer Isolation zwischen Nadelelektrode und Hülse sowie die Saug- und/oder Spülkanäle kurz vor dem zum Patienten proximalen Ende des Instruments in einem für den jeweiligen Verwendungszweck geeigneten Winkel abgebogen.

In einer Ausgestaltung der Erfindung ist die Hülse und/oder der Schaft des Instrumentes inclusive der Saug- und/oder Spülkanäle plastisch biegbar, so daß der Operateur das Instrument der jeweiligen anatomischen Situation anpassen kann.

Die zum Schneiden anwendbare Nadelelektrode kann manuell oder mittels eines elektromagnetischen, pneumatischen oder hydraulischen Antriebs zum Schutz gegen Verbiegen oder Abbrechen oder gegen unbeabsichtigte Verletzung des Patienten oder während Koagulationen, für die nur die Stirnfläche der Hülse angewendet wird, oder zum Saugen und/oder Spülen in die Hülse zurückgezogen und zum Schneiden wieder aus der Hülse herausgeschoben werden. Hierzu können auch mechanische Einrichtungen, wie sie beispielsweise in vielfältiger Ausführung bei Kugelschreibern zum rastenden Vor- und Rückführen bzw. zum Arretieren der Schreibmine bekannt sind, verwendet werden.

Der Antrieb für die Verschiebung der zum Schneiden anwendbare Nadelelektrode ist beispielsweise am vom Patienten aus gesehen distalen Ende des Schafts angeordnet, an welchem auch die elektrischen Anschlüsse für die zum Schneiden anwendbare Nadelelektrode und für die als Neutralelektrode oder Koagulationselektrode anwendbare Stirnfläche der Hülse sowie die Anschlüsse zum Saugen und/oder Spülen angeordnet sein können.

Die Erfindung wird im folgenden anhand schematischer Zeichnungen und Ausführungsbeispielen detaillierter beschrieben. Es zeigen:
**Figur 1** eine schematische Darstellung tumoröser Veränderungen im Rektum.
**Figur 2** eine schematische Darstellung einer Anwendung eines erfindungsgemäßen Instruments.
**Figur 3** eine Schnittdarstellung eines ersten Ausführungsbeispiels des zum Patienten proximalen Endes eines erfindungsgemäßen Instruments, und zwar gerade mit herausgeschobener Nadelelektrode in Figur 3a und gerade mit hineingezogener Nadelelektrode in Figur 3b sowie gebogen mit herausgeschobener Nadelelektrode in Figur 3c und gebogen mit hineingezogener Nadelelektrode in Figur 3d.
**Figur 4** eine Schnittdarstellung eines zweiten Ausführungsbeispiels des zum Patienten proximalen Endes eines erfindungsgemäßen Instruments, und zwar gerade mit herausgeschobener Nadelelektrode in Figur 4a und gerade mit hineingezogener Nadelelektrode in Figur 4b sowie gebogen mit herausgeschobener Nadelelektrode in Figur 4c und gebogen mit hineingezogener Nadelelektrode in Figur 4d.
**Figur 5** eine Schnittdarstellung eines dritten Ausführungsbeispiels des zum Patienten proximalen Endes des erfindungsgemäßen Instruments, und zwar gerade mit herausgeschobener Nadelelektrode in Figur 5a und gerade mit hineingezogener Nadelelektrode in Figur 5b sowie gebogen mit herausgeschobener Nadelelektrode in Figur 5c und gebogen mit hineingezogerner Nadelelektrode in Figur 5d.
**Figur 6** eine Schnittdarstellung eines vierten Ausführungsbeispiels des zum Patienten proximalen Endes eines erfindungsgemäßen Instruments, und zwar gerade mit herausgeschobener Nadelelektrode in Figur 6a und mit hineingezogener Nadelelektrode in Figur 6b sowie gebogen mit herausgeschobener Nadelelektrode in Figur 6c und gebogen mit hineingezogener Nadelelektrode in Figur 6d.
**Figur 7** eine schematische Darstellung eines Ausführungsbeispiels eines erfindungsgemäßen Instruments.
**Figur 8** eine schematische Darstellung verschiedener Möglichkeiten des Antriebs für die Schneideelektrode, und zwar eines elektromagnetischen Antriebs in Figur 8a und eines pneumatischen Antriebs in Figur 8b
**Figur 9** eine Ausgestaltung des erfindungsgemäßen Instruments. Hierbei ist der Schaft des Instruments plastisch biegbar gestaltet, so daß der Operateur das Instrument der jeweiligen anatomischen Situation anpassen kann.
**Figur 10** in schematischer Darstellung ein Operationsrektoskop.
**Figur 11** eine Schnittdarstellung eines fünften Ausführungsbeispiels des zum Patienten proximalen Endes eines erfindungsgemäßen Instruments, und zwar gerade mit herausgeschobener Nadelelektrode in Figur 11a und gerade mit hineingezogener Nadelelektrode in Figur 11b sowie gebogen mit herausgeschobener Nadelelektrode in Figur 11c und gebogen mit hineingezogener Nadelelektrode in Figur 11d.

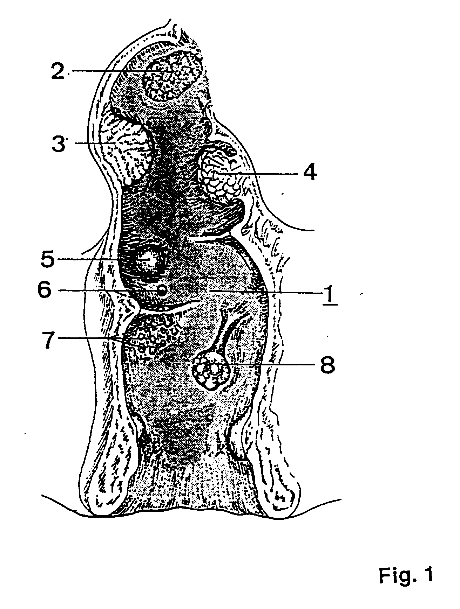

**Figur 1** zeigt Beispiele von tumorösen Veränderungen beispielsweise im Rektum 1, bei welchen ein erfindungsgemäßes Instrument beispielsweise angewendet werden kann, und zwar ulzerierende Karzinome 2, villöse Adenome 3, polypoide Karzinome 4, sessile adenomatöse Polypen 5, 6, 7 und/oder gestielte adenomatöse Polypen 8. Bei Anwendung von bisher bekannten Instrumenten können derartige tumoröse Veränderungen nicht so präzise und nicht ohne ständigem Wechseln von Schneide- und Koagulationsinstrumenten reseziert werden.

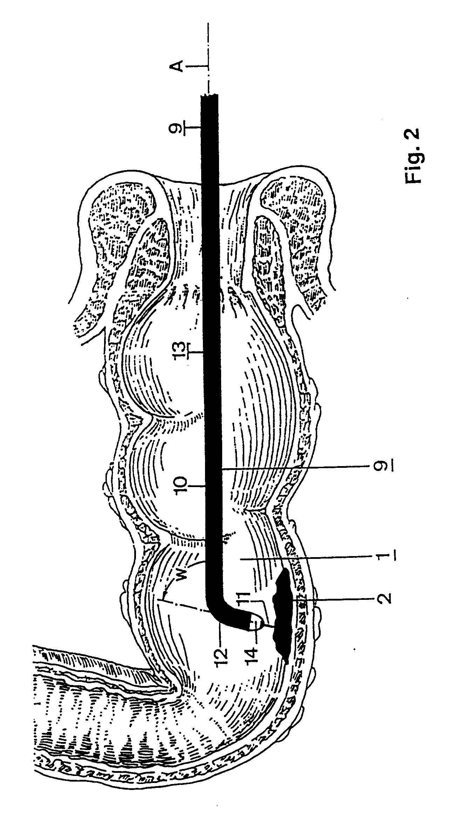

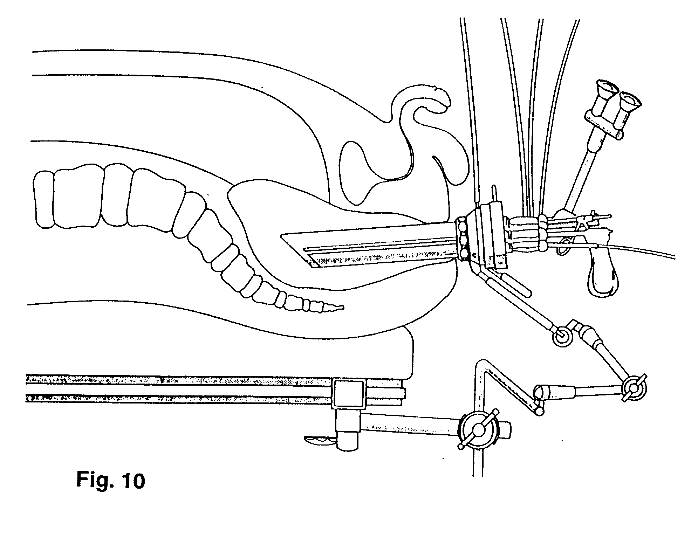

**Anhand von Figur 2** wird schematisch beispielsweise eine transanale Anwendung eines erfindungsgemäßen Instruments zur operativen Therapie eines Karzinoms im Rektum 1 dargestellt, wobei mit Rücksicht auf eine übersichtliche Darstellung nur das zum Patienten proximale Teil des Instruments 9 dargestellt ist. Das Instrument 9 ist transanal ins Rektum 1 eingeführt. In der Praxis erfolgt die Anwendung des Instruments durch ein geeignetes Operationsrektoskop, wie es in Figur 10 schematisch dargestellt ist. Das Instrument 9 ist bis zum abzutragenden Kartinom 2 eingeführt. Zum Resezieren des Karzinoms 2 ist die Nadelelektrode 11 aus dem Instrument 9 herausgeschoben. Wird während der Resektion ein Blutgefäß perforiert, so kann es mit dem als Koagulationselektrode anwendbaren Stirnfläche 14 des zum Patienten proximalen Endes der Hülse 12 bzw. des Instruments 9 koaguliert werden. Während einer Koagulation kann die Nadelelektrode 11 in das Instrument 9 zurückgezogen sein. Das zum Patienten proximale Ende der Hülse 12 bzw. des Instruments 9 ist gegen die Achse A des Schafts 13 in einem für den jeweiligen Anwendungszweck geeigneten Winkel W abgebogen. Der Schaft 13 und die Hülse 12 sind auf ihrer Außenfäche, exklusive der als Koagulationselektrode ausgebildeten konvexen Stirnfläche 14, mit einer elektrischen Isolation 10 ausgestattet.

Das erfindungsgemäße Instrument kann auf verschiedene Weisen betrieben bzw. an einen Hochfrequenzgenerator angeschlossen werden. So kann die Nadelelektrode 11 als monopolare Schneideelektrode betrieben werden, wobei als Gegenelektrode eine bei monopolaren Techniken übliche, bekannte Neutralelektrode in bekannter Weise auf der Haut des Patienten appliziert ist. Hierbei wird die Nadelelektrode 11 an die monopolare, aktive Ausgangsbuchse und die Neutralelektrode an die neutrale Ausgangsbuchse des Hochfrequenzgenerators angeschlossen. Als Gegenelektrode kann aber auch die Stirnfläche 14 der Hülse 12 verwendet werden, wie es in US-A-4,043,342 beschrieben ist. Hierbei wird die Nadelelektrode 11 an die monopolare, aktive Ausgangsbuchse und die Stirnfläche 14 der Hülse 12 an die neutrale Ausgangsbuchse des Hochfrequenzgenerators angeschlossen.

Die Stirnfläche 14 der Hülse 12 kann als monopolare Koagulationselektrode betrieben werden, wobei als Gegenelektrode eine bei monopolaren Techniken übliche, bekannte Neutralelektrode in bekannter Weise auf der Haut des Patienten appliziert ist. Hierbei wird die als Koagulationselektrode dienende Stirnfläche 14 der Hülse 12 an die monopolare, aktive Ausgangsbuchse und die aufder Haut des Patienten applizierte Neutralelektrode an der neutralen Ausgangsbuchse des Hochfrequenzgenerators angeschlossen.

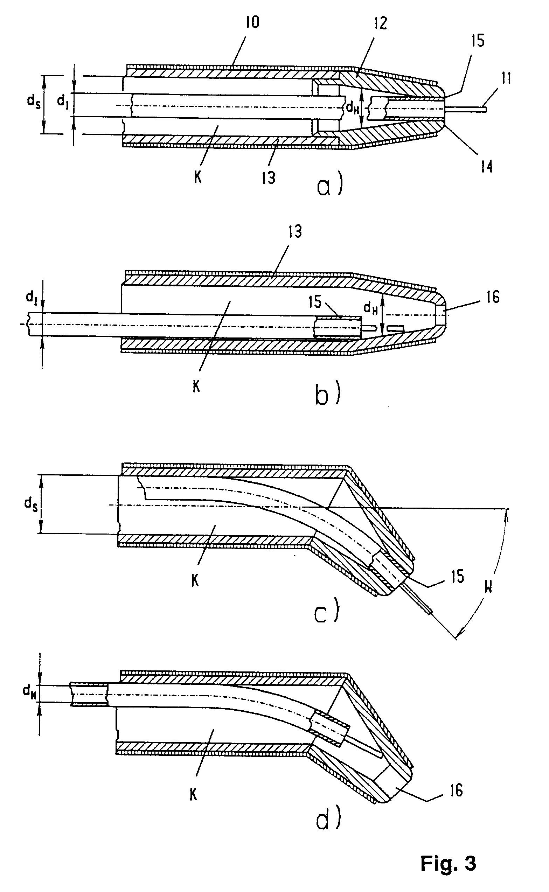

**In Figur 3** ist das zum Patienten proximale Ende eines erfindungsgemäßen Instruments 9 im Schnitt dargestellt, und zwar gerade mit herausgeschobener Nadelelektrode 11 zum Schneiden in Figur 3a und gerade mit hineingezogener Nadelelektrode 11 zum Koagulieren, Saugen und/oder Spülen oder in Ruhestellung in Figur 3b sowie gebogen mit herausgeschobener Nadelelektrode zum Schneiden in Figur 3c und gebogen mit hineingezogener Nadelelektrode zum Koagulieren, Saugen und/oder Spülen oder in Ruhestellung in Figur 3d.

Das zum Patienten proximale Ende dieses erfindungsgemäßen Instruments besteht aus einer metallischen, vorzugsweise runden Hülse 12, welche durch einen vorzugsweise runden, metallischen Schaft 13 verlängert ist. Hülse 12 und Schaft 13 können auch einstückig aus einem metallischen Rohr geformt sein, wie es in Figur 3b dargestellt ist. Der Schaft 13 kann eine derartige Länge haben, daß das Instrument auch für endoskopische Anwendungen geeignet ist. Hülse 12 und Schaft 13 sind auf ihrer äußeren Oberfläche exclusive der Stirnfläche 14 der Hülse 12, welche als Koagulationselektrode oder beim Schneiden mit der Nadelelektrode 11 als Neutralelektrode angewendet werden kann, mit einer elektrischen Isolierschicht 10 ausgestattet. In der Stirnfläche 14 der Hülse 12 ist ein Loch 16 vorhanden, durch welches die Nadelelektrode 11 hindurchschiebbar ist. Der Durchmesser dieses Lochs 16 ist so groß, daß die Isolation 15 der Nadelelektrode 11 einerseits leicht durch dieses Loch hindurchgeschoben werden kann und andererseits so eng, daß die Nadelelektrode 11 von der Hülse 12 ohne zu wackeln gut geführt wird.

Der Innendurchmesser d_{H} der Hülse 12 und/oder d_{S} des Schafts 13 bzw. des Instruments 9 ist derart groß, daß zwischen dem Außendurchmesser d_{I} der Isolation 15 der Nadelelektrode 11 und dem Innendurchmesser d_{H} der Hülse 12 und d_{S} des Schafts ein Kanal K zum Saugen und/oder Spülen vorhanden ist.

Die Außendurchmesser der Hülse 12 und des Schaftes 13 können prinzipiell gleich sein. Mit Rücksicht auf eine gute Sicht auf die Nadelelektrode 11 und eine nicht zu große effektive Kontaktfläche der als Koagulationselektrode oder beim Schneiden mittels der Nadelelektrode 11 als Neutralelektrode anwendbaren, vorzugsweise konvexen Stirnfläche 14 der Hülse 12 ist es jedoch zweckmäßig, den Außendurchmesser der Hülse 12 in der Nähe der Stirnfläche 14 möglichst klein, beispielsweise 3 bis 5 Millimeter zu wählen. Mit Rücksicht auf eine gute mechanische Stabilität des Instruments und einen möglichst großen Querschnitt des Saug- und/oder Spülkanals K ist es zweckmäßig, den Außendurchmesser des Schafts 13 nicht zu klein zu wählen, beispielsweise 5 bis 10 Millimeter. Der Außendurchmesser der Hülse 12 kann zweckmäßig in mindestens einer Stufe und/oder konisch, wie in Figur 3 dargestellt, vom Außendurchmesser des Schaftes 13 auf den Durchmesser der Stirnfläche 14 reduziert werden.

Die Nadelelektrode 11 ist mit einer elektrischen Isolation 15 gegen die metallische Hülse 12 und den Schaft 13 isoliert.

Die Nadelelektrode 11 kann, wie in Figur 3b und 3d dargestellt, soweit in die Hülse 12 zurückgezogen werden, daß das Loch 16 in der Stirnfläche 14 zum Saugen und/oder Spülen frei ist.

Um zu gewährleisten, daß die zum Schneiden effektive Länge der aus der Stirnfläche 14 der Hülse 12 herausschiebbaren Nadelelektrode 11 möglichst genau und reproduzierbar einstellbar ist, muß die relativ dünne Nadel 11 entweder so innerhalb des relativ großlumigen Saug- und/oder Spülkanals geführt werden, daß sie sich innerhalb dieses Kanals nicht verbiegen kann, beispielsweise indem sie innerhalb des Kanals K gestützt wird, oder sie muß, wie beispielsweise bei den Ausführungsbeispielen in den Figuren 3a bis 3d dargestellt, innerhalb des Instruments auf den Durchmesser d_{N} verstärkt werden.

Je nach Anwendungsgebiet kann es zweckmäßig sein, das Instrument gerade bzw. axial auszurichten, wie es beispielsweise in den Ausführungsbeispielen der Figuren 3a und 3b dargestellt ist, oder das zum Patienten proximale Ende der Hülse 12 und/oder des Schafts 13 bzw. des Instruments in einem für den jeweiligen Verwendungszweck geeigneten Winkel W abzubiegen, wie es beispielsweise in den Figuren 3c und 3d dargestellt ist.

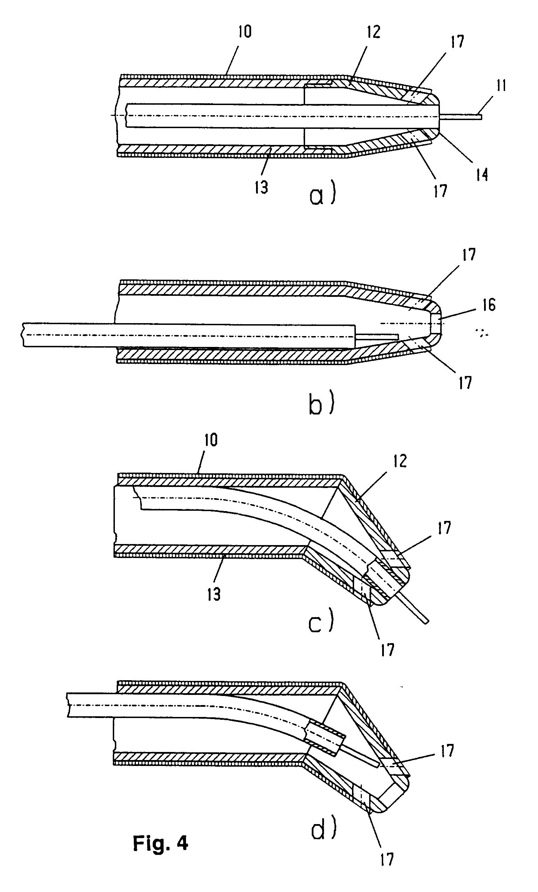

**Figur 4** zeigt eine Schnittdarstellung eines zweiten Ausführungsbeispiels des zum Patienten proximalen Endes eines erfindungsgemäßen Instruments, und zwar gerade mit herausgeschobener Nadelelektrode in Figur 4a und gerade mit hineingezogener Nadelelektrode in Figur 4b sowie gebogen mit herausgeschobener Nadelelektrode in Figur 4c und gebogen mit hineingezogener Nadelelektrodein Figur 4d.

Dieses Ausführungsbeispiel unterscheidet sich zu dem Ausführungsbeispiel in den Figuren 3a bis 3d durch zusätzliche Löcher 17 zum Saugen und/oder Spülen in unmittelbarer Nähe zur Stirnfläche 14 der Hülse 12, welche vorzugsweise radial derart um das Loch 16 herum angeordnet sind, daß Saugen und/oder Spülen durch diese zusätzlichen Löcher 17 auch dann möglich ist, wenn die Nadelelektrode 11 aus dem Loch 16 herausgeschoben ist oder wenn die Nadelelektrode in die Hülse hineingezogen ist, das Loch 16 jedoch gegen Gewebe gedrückt wird, wie es beispielsweise während Koagulationen der Fall ist. Diese zusätzlichen Löcher 17 können auch zum Absaugen des während Scheide- und/oder Koagulationsvorgängen enstehenden Dampfs und/oder Rauchs angewendet werden.

Hülse 12 und Schaft 13 können beispielsweise aus zwei Teilen zusammengefügt sein, wie es in Figur 4a dargestellt , oder sie können einstückig aus einem Rohrgeformt sein, wie es in Figur 4b dargestellt ist.

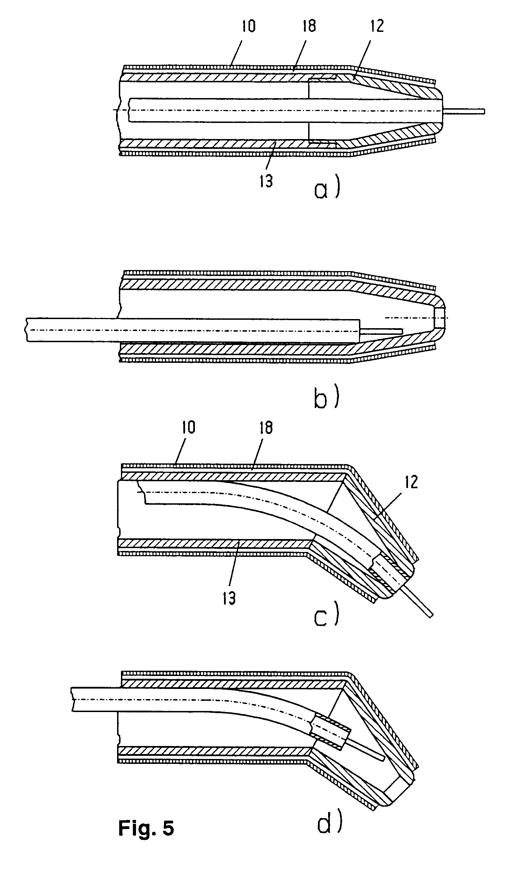

**Figur 5** zeigt eine Schnittdarstellung eines dritten Ausführungsbeispiels des zum Patienten proximalen Endes eines erfindungsgemäßen Instruments, und zwar gerade mit herausgeschobener Nadelelektrode in Figur 5a und gerade mit hineingezogener Nadelelektrode in Figur 5b sowie gebogen mit herausgeschobener Nadelelektrode in Figur 5c und gebogen mit hineingezogerner Nadelelektrode in Figur 5d.

Dieses Ausführungsbeispiel unterscheidet sich zu dem Ausführungsbeispiel der Figuren 3a bis 3d durch einen zusätzlichen Saug- und/oder Spülkanal 18, der beispielsweise konzentrisch zwischen den Außendurchmessern der Hülse 12 und des Schafts 13 und der Isolationsschicht 10 eingefügt ist. Die Distanz zwischen den Oberflächen von Hülse und Schaft einerseits und Isolationsschicht andererseits kann beispielsweise durch Noppen oder axiale Profile auf den Oberfächen von Hülse und Schaft oder der Innenfläche der Isolation 10 realisiert werden.

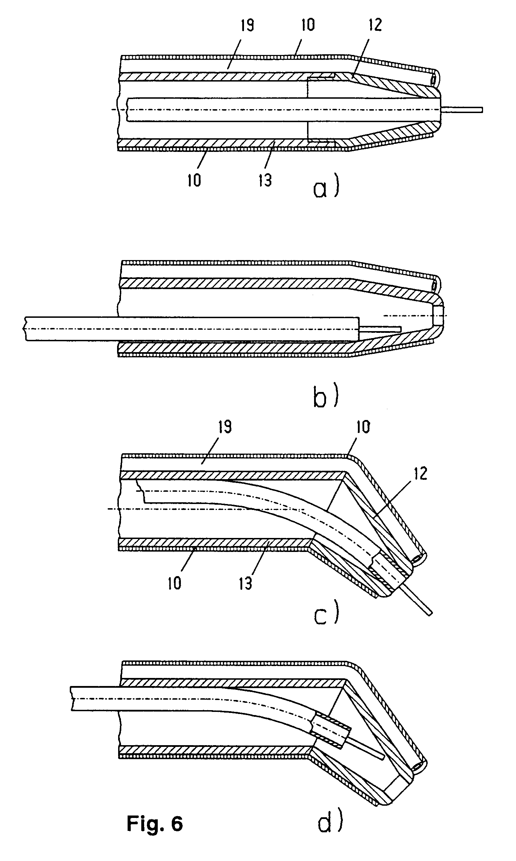

**Figur 6 zeigt** eine Schnittdarstellung eines vierten Ausführungsbeispiels des zum Patienten proximalen Endes eines erfindungsgemäßen Instruments, und zwar gerade mit herausgeschobener Nadelelektrode in Figur 6a und gerade mit hineingezogener Nadelelektrode in Figur 6b sowie gebogen mit herausgeschobener Nadelelektrode in Figur 6c und gebogen mit hineingezogener Nadelelektrode in Figur 6d.

Dieses Ausführungsbeispiel unterscheidet sich von dem in Figur 3 dargestellten Ausführungsbeispiel durch einen zusätzlichen Saug- und/oder Spülkanal, der beispielsweise durch ein Rohr 19 realisiert ist, welches parallel zur Hülse 12 und zum Schaft 13 geführt ist und beispielsweise auf der Oberfläche von Hülse und Schaft aufgelötet ist. Besteht dieses Rohr 19 aus Metall, so muß es wie die Hülse 12 und der Schaft 13 auf seiner Oberfläche mit einer elektrischen Isolierschicht 10 ausgestattet sein. Das Rohr 20 endet vorzugsweise kurz vor der Stirnfläche 14 der Hülse 12.

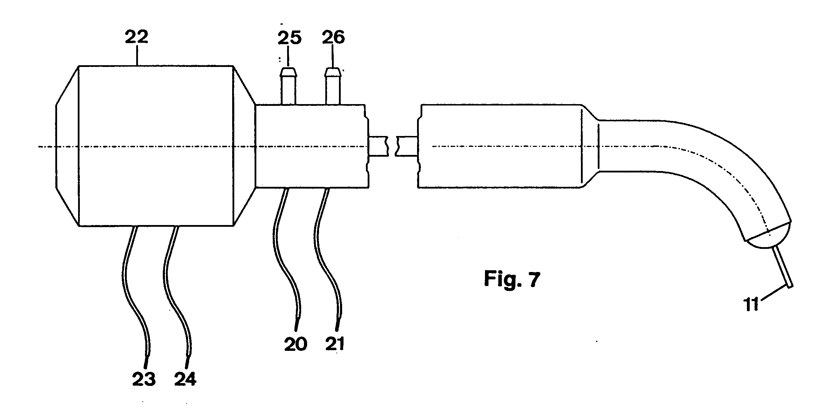

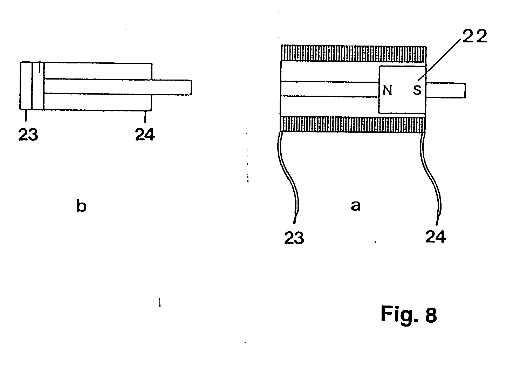

**In Figur 7** ist schematisch ein Ausführungsbeispiel eines erfindungsgemäßen Instruments dargestellt. Am vom Patienten distalen Ende des Instruments ist ein Antrieb 22 zum Herausschieben bzw. Hineinziehen der Nadelelektrode 11 schematisch dargestellt. Dieser Antrieb 22 kann beispielsweise elektromagnetisch, wie in **Figur 8a** schematisch dargestellt, oder pneumatisch oder hydraulisch, wie in **Figur 8b** schematisch dargestellt, oder mechanisch, wie beispielsweise bei Kugelschreibern zum Heraus- und Hineinbewegen und Arretieren der Schreibmine bekannt, realisiert werden. Die Anschlüsse 23 und 24 sind entweder Elektroanschlüsse für einen elektromagnetischen Antrieb 22 oder Druckluftanschlüsse für einen pneumatischen Antrieb 22 oder Schlauchanschlüsse für einen hydraulichen Antrieb 22. Die elektrischen Anschlüsse 20 und 21 dienen zur HF-Stromversorgung der Nadelelektrode 11 und der als Neutralelektrode oder Koagulationselektrode anwendbaren Stirnfläche 14 der Hülse 12. 25 und 26 sind Anschlüsse für Saug- und/oder Spülleitungen um das Instrument mit Saug- und/oder Spüleinrichtungen zu verbinden.

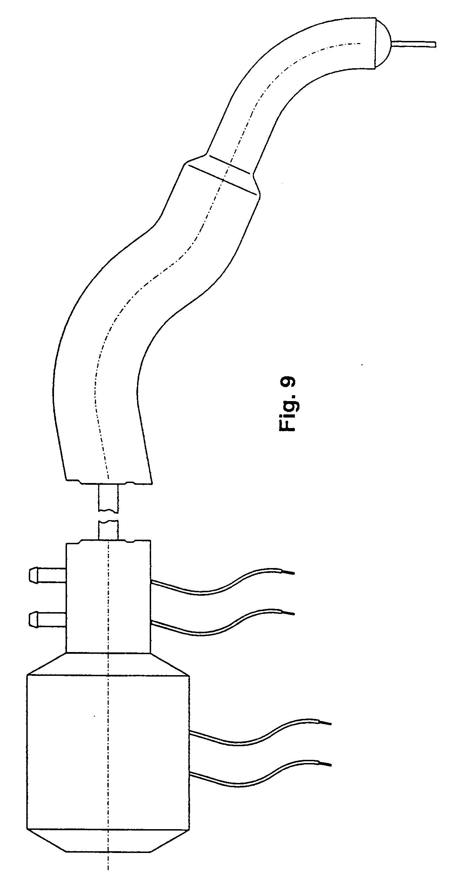

**In Figur 9** ist ein erfindungsgemäßes Instrument schematisch dargestellt, bei welchem der Schaft 13 vom Operateur plastisch so gebogen werden kann, daß es den individuellen bzw. anatomischen Verhältnissen angepaßt werden kann.

**Figur 10** zeigt in schematischer Darstellung ein Operationsrektoskop zur transanalen endoskopischen Resektion tumoröser Veränderungen im Rektum, durch welches hindurch das erfindungsgemäße Instrument beispielsweise angewendet werden kann.

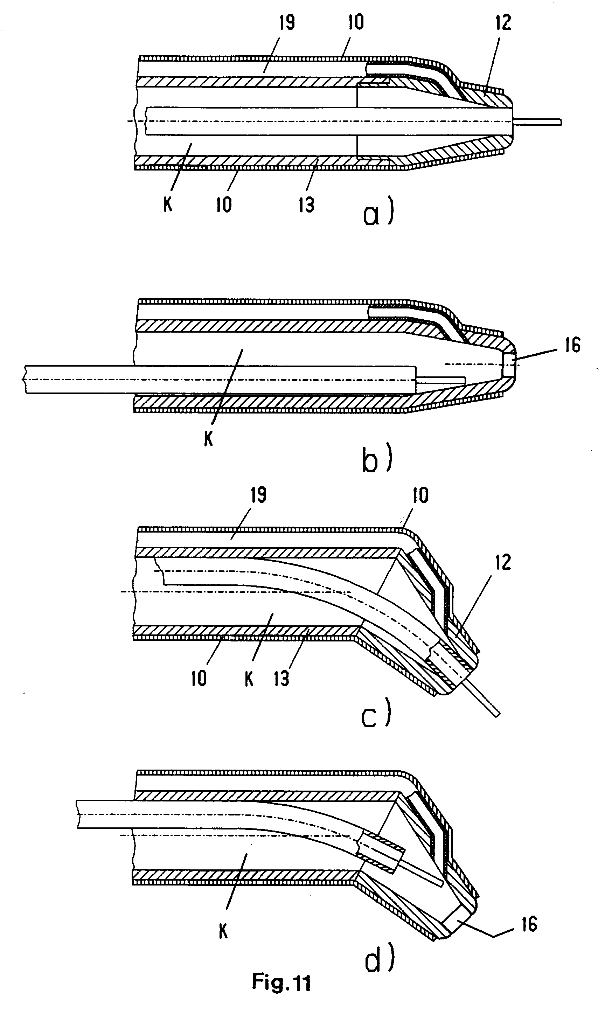

**Figur 11** eine Schnittdarstellung eines fünften Ausführungsbeispiels des zum Patienten proximalen Endes eines erfindungsgemäßen Instruments, und zwar gerade mit herausgeschobener Nadelelektrode in Figur 11a und gerade mit hineingezogener Nadelelektrode in Figur 11b sowie gebogen mit herausgeschobener Nadelelektrode in Figur 11c und gebogen mit hineingezogener Nadelelektrode in Figur 11d.

Bei dieser Ausführung eines erfindungsgemäßen Instruments sind zwei Kanäle K; 19 vorhanden, welche innerhalb und/oder außerhalb des Schafts 13 und/oder der Hülse 12, voneinander getrennt bis zur Hülse 12 und/oder bis kurz vor die Saug- und/oder Spüllöffnung 16 geführt werden, wo sie innerhalb des Instruments vereinigt in die gemeinsame Öffnung 16 einmünden, durch welche abwechselnd Saugen oder Spülen möglich ist. Bei einer derartigen Ausführung ergibt sich zusätzlich der Vorteil, daß eine einfache Reinigung des Saugkanals möglich ist, indem die gemeinsame Öffnung 16 beispielsweise durch Herausschieben der Nadelelektrode 11 geschlossen wird und die Spülflüssigkeit innerhalb des Instruments kurz vor der geschlossenen Öffnung 16 aus dem Spülkanal 19 in den Saugkanal K hineingesaugt wird um den Saugkanal K beispielsweise von Blut und anderen Gewebebestandteile zu reinigen. Das Instrument ist, beispielsweise für endoskopische Anwendungen, auf seiner Außenfläche, mit Ausnahme der als Neutralelektrode oder Koagulationselektrode dienenden, zum Patienten proximalen Stirnfläche 14 , elektrisch isoliert.

## Patentansprüche

1. Instrument für die Hochfrequenzchirurgie zum Schneiden und/oder Koagulieren biologischer Gewebe, mit einer als Neutralelektrode dienenden Hülse (12), in welcher eine als Schneidelektrode dienende, gegen die Hülse elektrisch isolierte Nadel (11) aus Metall durch eine Öffnung (16) in der Hülse (12) in axialer Richtung verschiebbar angeordnet ist, wobei innerhalb der Hülse (12) ein mit einem Anschluß (25; 26) zum Saugen und/oder Spülen versehener Kanal (K) vorgesehen ist,
**dadurch gekennzeichnet,**
daß die als Schneidelektrode anwendbare Nadel (11) von einer die Öffnung (16) verschließenden Position so weit zurückziehbar ist, daß diese Öffnung (16) in der Stirnfläche (14) der Hülse (12) als Mündung zum Saugen und/oder Spülen verwendbar ist.

2. Instrument nach Anspruch 1,
**dadurch gekennzeichnet,**
daß eine zusätzliche Öffnung zum Saugen und/oder Spülen in der Stirnfläche der Hülse (12) vorhanden ist, durch welche Saugen und/oder Spülen auch dann möglich ist, wenn die Nadelelektrode aus ihrer Öffnung (16) nach außen hervorragend, die Öffnung verschließend, herausgeschoben ist, oder wenn die Nadelelektrode (11) in die Hülse zurückgezogen ist und ihre Öffnung (16) gegen Gewebe gedrückt wird, wobei die zusätzliche Öffnung in einen Kanal (19) übergeht, welcher in Längsrichtung auf der Außenseite der Hülse (12) verläuft oder die Hülse (12) konzentrisch umgibt.

3. Instrument nach Anspruch 2,
**dadurch gekennzeichnet,**
daß die Öffnung als konzentrischer Spalt (18) zwischen Hülse (12) und einer die Hülse (12) umgebenden Isolierschicht (10) ausgebildet ist.

4. Instrument nach einem der vorangegangenen Ansprüche,
**dadurch gekennzeichnet,**
daß die Hülse (12) einen angrenzenden Schaft (13) aufweist, wobei Schaft und Hülse eine für endoskopische Operationen geeignete Länge besitzen, wobei das zum Patienten proximale Ende des Instrumentes axial ausgerichtet oder um einen Winkel (W) aus der Achse (A) des Instrumentes abgebogen ist.

5. Instrument nach einem der vorangegangenen Ansprüche,
**dadurch gekennzeichnet,**
daß eine Antriebseinrichtung und eine Arretiereinrichtung vorhanden sind, mit deren Hilfe die zum Schneiden geeignete Nadelelektrode (11) in die Hülse (12) hineingezogen oder aus der Hülse (12) herausgeschoben und in vorgegebenen Positionen arretierbar ist.

6. Instrument nach einem der vorangegangenen Ansprüche,
**dadurch gekennzeichnet,**
daß die Nadelelektrode (11) als monopolare Schneidelektrode an ein Hochfrequenz-Chirurgiegerät anschließbar ist, wobei als Gegenelektrode eine bei monopolaren Techniken übliche Neutralelektrode in an sich bekannter Weise auf der Haut des Patienten applizierbar und am Hochfrequenz-Chirurgiegerät anschließbar sen ist.

7. Instrument nach einem der Ansprüche 1 bis 5,
**dadurch gekennzeichnet,**
daß die Nadelelektrode als monopolare Schneidelektrode und die Stirnfläche (14) der Hülse als Neutralelektrode an ein Hochfrequenz-Chirurgiegrät anschließbar sind.

8. Instrument nach einem der Ansprüche 1 bis 5,
**dadurch gekennzeichnet,**
daß die Nadelelektrode als monopolare Schneidelektrode und die Stirnfläche (14) der Hülse (12) als monopolare Koagulationselektrode an ein Hochfrequenz-Chirurgiegerät anschließbar sind, wobei als Gegenelektrode eine bei monopolaren Techniken übliche Neutralelektrode in an sich bekannter Weise auf der Haut des Patienten applizierbar und am Hochfrequenz-Chirurgiegerät anschließbar ist.

9. Instrument nach Anspruch 5,
**dadurch gekennzeichnet,**
daß die Antriebseinrichtung (22) elektromagnetisch, pneumatisch oder hydraulisch betätigbar ist.

10. Instrument nach einem der Ansprüche 4 bis 9,
**dadurch gekennzeichnet,**
daß Hülse (12) und Schaft (13) einstückig ausgebildet sind.

11. Instrument nach einem der Ansprüche 4 bis 10,
**dadurch gekennzeichnet,**
daß die Hülse (12) und der Schaft (13) gleiche Außendurchmesser aufweisen.

12. Instrument nach einem der Ansprüche 4 bis 11,
**dadurch gekennzeichnet,**
daß der Außendurchmesser der Hülse (12) in der Nähe der Stirnfläche (14) kleiner als der Außendurchmesser des Schaftes (13) ist.

13. Instrument nach Anspruch 12,
**dadurch gekennzeichnet,**
daß der Außendurchmesser des Schaftes (13) im Bereich zwischen 5 und 10 mm, der Außendurchmesser der Stirnfläche (14) der Hülse zwischen 2 und 5 mm und der Durchmesser der Nadelelektrode (11) im Bereich zwischen 0,1 und 0,5 mm liegt.

14. Instrument nach Anspruch 12 oder 13,
**dadurch gekennzeichnet,**
daß der Außendurchmesser des Schaftes (13) in mindestens einer Stufe oder konisch auf einen kleineren Außendurchmesser der Stirnfläche (14) der Hülse (12) reduziert ist.

15. Instrument nach Anspruch 4,
**dadurch gekennzeichnet,**
daß der Schaft (13) plastisch biegbar ist.

## Claims

1. An instrument for high frequency surgery for the cutting and/or coagulating of biological tissue, with a casing (12) serving as a neutral electrode, in which a needle (11) of metal, serving as cutting electrode and insulated electrically with respect to the casing, is arranged so as to be displaceable in axial direction through an opening (16) in the casing (12), in which within the casing (12) a channel (K) is provided which is provided with a connection (25; 26) for sucking and/or flushing,
characterised in that
the needle (11) which can be used as a cutting electrode, can be drawn back from a position closing the opening (16) so far that this opening (16) in the end face (14) of the casing (12) can be used as an orifice for sucking and/or flushing.

2. An instrument according to Claim 1, characterised in that
an additional opening for sucking and/or flushing is present in the end face of the casing (12), through which sucking and/or flushing is also possible when the needle electrode, projecting outwards from its opening (16), is pushed-out closing the opening, or when the needle electrode (11) is drawn back into the casing and its opening (16) is pressed against tissue, in which the additional opening continues into a channel (19) which runs in longitudinal direction on the outer side of the casing (12) or surrounds the casing (12) concentrically.

3. An instrument according to Claim 2,
characterised in that
the opening is constructed as a concentric gap (18) between the casing (12) and an insulating layer (10) surrounding the casing (12).

4. An instrument according to one of the preceding claims,
characterised in that
the casing (12) has an adjoining shaft (13), in which the shaft and the casing have a suitable length for endoscopic operations, in which the end of the instrument proximal to the patient is axially aligned or is bent about an angle (W) from the axis (A) of the instrument.

5. An instrument according to one of the preceding claims,
characterised in that
a driving device and an arresting device are present, by means of which the needle electrode (11), which is suitable for cutting, is drawn into the casing (12) or pushed out from the casing (12) and can be arrested in given positions.

6. An instrument according to one of the preceding claims,
characterised in that the needle electrode (11) as a monopolar cutting electrode is connectable to a high frequency surgical apparatus, in which as counter electrode a neutral electrode, which is usual in monopolar techniques, can be applied to the skin of the patient in a manner known per se and is connectable to the high frequency surgical apparatus.

7. An instrument according to one of Claims 1 to 5,
characterised in that
the needle electrode as monopolar cutting electrode and the end face (14) of the casing as neutral electrode are connectable to a high frequency surgical apparatus.

8. An instrument according to one of Claims 1 to 5,
characterised in that
the needle electrode as monopolar cutting electrode and the end face (14) of the casing (12) as monopolar coagulation electrode are connectable to a high frequency surgical apparatus, in which as counter electrode a neutral electrode which is usual in monopolar techniques can be applied to the skin of the patient in a manner known per se and is connectable to the high frequency surgical apparatus.

9. An instrument according to Claim 5,
characterised in that
the drive device (22) can be actuated electromagnetically, pneumatically or hydraulically.

10. An instrument according to one of Claims 4 to 9,
characterised in that
the casing (12) and shaft (13) are constructed in one piece.

11. An instrument according to one of Claims 4 to 10,
characterised in that
the casing (12) and the shaft (13) have the same external diameters.

12. An instrument according to one of Claims 4 to 11,
characterised in that
the external diameter of the casing (12) in the vicinity of the end face (14) is smaller than the external diameter of the shaft (13).

13. An instrument according to Claim 12, characterised in that
the external diameter of the shaft (13) lies in the range between 5 and 10 mm, the external diameter of the end face (14) of the casing lies between 2 and 5 mm and the diameter of the needle electrode (11) lies in the range between 0.1 and 0.5 mm.

14. An instrument according to Claim 12 or 13,
characterised in that
the external diameter of the shaft (13) is reduced in at least one step or conically to a smaller external diameter of the end face (14) of the casing (12).

15. An instrument according to Claim 4,
characterised in that
the shaft (13) can be bent in a plastic manner.

## Revendications

1. Instrument pour la chirurgie à haute fréquence pour la découpe et/ou la coagulation de tissus biologiques, équipé d'un manchon (12) servant d'électrode neutre, dans lequel une aiguille (11) en métal servant d'électrode de découpage et isolée électriquement par rapport au manchon est disposée de façon coulissante dans le sens axial à travers une ouverture (16) pratiquée dans le manchon (12), un canal (K) pourvu d'un raccordement (25 ; 26) pour l'aspiration et/ou le lavage étant prévu à l'intérieur du manchon (12), caractérisé en ce que l'aiguille (11) utilisable comme électrode de découpe peut être retirée d'une position verrouillant l'ouverture (16) assez largement pour que cette ouverture (16) pratiquée dans la surface frontale (14) du manchon (12) puisse être utilisée comme orifice pour l'aspiration et/ou le lavage.

2. Instrument selon la revendication 1, caractérisé en ce qu'il existe une ouverture supplémentaire pour l'aspiration et/ou le lavage de la surface frontale du manchon (12) qui permet l'aspiration et/ou le lavage également dans les cas où l'électrode-aiguille est sortie, par poussée, de son ouverture (16), en dépassant vers l'extérieur et en fermant l'ouverture, ou lorsque l'électrode-aiguille (11) est reculée dans le manchon et que son ouverture (16) est appuyée contre le tissu, l'ouverture supplémentaire se transformant en un canal (19) qui va dans le sens longitudinal sur la face externe du manchon (12) ou entoure le manchon (12) de façon concentrique.

3. Instrument selon la revendication 2, caractérisé en ce que l'ouverture est conçue comme une lente (18) concentrique entre le manchon (12) et une couche isolante (10) entourant le manchon (12).

4. Instrument selon l'une quelconque des revendications précédentes, caractérisé en ce que le manchon (12) présente un axe (13) limitrophe, l'axe et le manchon ayant une longueur appropriée pour des opérations endoscopiques, l'extrémité de l'instrument la plus rapprochée du patient étant orientée dans le sens axial ou pliée d'un angle (W) par rapport à l'axe (A) de l'instrument.

5. Instrument selon l'une quelconque des revendications précédentes, caractérisé en ce qu'il existe un dispositif d'entraînement et un dispositif d'arrêt à l'aide desquels l'électrode-aiguille (11) appropriée pour la découpe est rentrée dans le manchon (12) ou poussée hors du manchon (12) et peut être bloquée dans des positions prédéfinies.

6. Instrument selon l'une quelconque des revendications précédentes, caractérisé en ce que l'électrode-aiguille (11) peut être raccordée en tant qu'électrode monopolaire de découpage à un appareil de chirurgie à haute fréquence, une électrode neutre classique avec des techniques monopolaires pouvant être appliquées comme contre-électrode de façon connue en soi sur la peau du patient et pouvant être raccordée à l'appareil chirurgical à haute fréquence.

7. Instrument selon l'une quelconque des revendications 1 à 5, caractérisé en ce que l'électrode-aiguille et la surface frontale (14) du manchon peuvent être raccordées respectivement en guise d'électrode monopolaire de découpage et d'électrode neutre à un appareil chirurgical à haute fréquence.

8. Instrument selon l'une quelconque des revendications 1 à 5, caractérisé en ce que l'électrode-aiguille et la surface frontale (14) du manchon (12) peuvent être raccordées respectivement en guise d'électrode monopolaire de découpage et d'électrode monopolaire de coagulation à un appareil chirurgical à haute fréquence, une électrode neutre classique avec des techniques monopolaires pouvant être appliquée en guise de contre-électrode de façon connue en soi sur la peau du patient et pouvant être raccordée à l'appareil chirurgical a haute fréquence.

9. Instrument selon la revendication 5, caractérise en ce que le dispositif d'entraînement (22) peut être actionné de façon électromagnétique, pneumatique ou hydraulique.

10. Instrument selon l'une quelconque des revendications 4 à 9, caractérisé en ce que le manchon (12) et l'axe (13) sont réalisés d'un seul tenant.

11. Instrument selon l'une quelconque des revendications 4 à 10, caractérisé en ce que le manchon (12) et l'axe (13) ont des diamètres extérieurs identiques.

12. Instrument selon l'une quelconque des revendications 4 à 11, caractérisé en ce que le diamètre extérieur du manchon (12) est inférieur à proximité de la surface frontale (14) au diamètre extérieur de l'axe (13).

13. Instrument selon la revendication 12, caractérisé en ce que le diamètre extérieur de l'axe (13) se situe dans une plage comprise entre 5 et 10 mm, le diamètre extérieur de la surface frontale (14) du manchon entre 2 et 5 mm et le diamètre de l'électrode-aiguille (11) dans une plage comprise entre 0,1 et 0,5 mm.

14. Instrument selon la revendication 12 ou 13, caractérisé en ce que le diamètre extérieur de l'axe (13) est réduit sur au moins un niveau ou de façon conique jusqu'à un diamètre extérieur inférieur de la surface frontale (14) du manchon (12).

15. Instrument selon la revendication 4, caractérisé en ce que l'axe (13) peut être plié de façon plastique.
